(19) **Europäisches Patentamt / European Patent Office / Office européen des brevets**

(11) **EP 4 082 548 A2**

(12) # EUROPEAN PATENT APPLICATION
published in accordance with Art. 153(4) EPC

(43) Date of publication:
**02.11.2022 Bulletin 2022/44**

(21) Application number: **20912369.4**

(22) Date of filing: **18.12.2020**

(51) International Patent Classification (IPC):
**A61K 31/5513** [(2006.01)]     **A61P 25/28** [(2006.01)]

(52) Cooperative Patent Classification (CPC):
**A61K 31/5513; A61P 25/28**

(86) International application number:
**PCT/CU2020/050009**

(87) International publication number:
**WO 2021/139842 (15.07.2021 Gazette 2021/28)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.12.2019 CU 20190115**

(71) Applicant: **Centro de Investigación y Desarrollo de Medicamentos CIDEM**
**10400 la Habana (CU)**

(72) Inventors:
- **VIEIRA FURTADO, Andrezza Bond**
  **97050550 Santa Maria (BR)**
- **ALVES COURTES, Aline**
  **97015110 Santa Maria (BR)**
- **FARINA GONÇALVES, Debora**
  **97105430 Santa Maria (BR)**
- **DUARTE HARTMANN, Diane**
  **97060470 Santa Maria (BR)**
- **CASSOL, Gustavo**
  **97220000 Santa Maria (BR)**
- **FREIRE ROYES, Luiz Fernando**
  **97110633 Santa Maria (BR)**
- **ANTUNES SOARES, Félix Alexandre**
  **97105-260 Santa Maria (BR)**
- **NÚÑEZ FIGUEREDO, Yanier**
  **14000 La Habana (CU)**
- **MONDELO RODRÍGUEZ, Abel**
  **19270 La Habana (CU)**
- **PADRÓN YAQUIS, Alejandro Saúl**
  **Playa La Habana (CU)**

(74) Representative: **Capitán García, Maria Nuria**
**Felipe IV no. 10, bajo iz.**
**28014 Madrid (ES)**

(54) **USE OF A BENZODIAZEPINE DERIVATIVE AND METHOD OF TREATMENT OF TRAUMATIC BRAIN INJURY**

(57)     Present invention aims to protect a new use of JM-20, a benzodiazepine derivative, in the treatment of and recovery from traumatic brain injury (TBI) and its related symptoms. Additionally, it provides a method of treatment for TBI-induced brain damage.

EP 4 082 548 A2

**Description**

Field of the Invention

This invention relates to the field of healthcare; particularly with a new use of compound JM-20, a benzodiazepine derivative. Present invention specifically proposes the use of JM-20 in the treatment of and recovery from traumatic brain injury (TBI) and related symptoms.

Background of the invention

TBI occurs when an external mechanic force causes brain dysfunction. TBI is characterized by a sudden physical damage to the brain. It can be caused by several factors, including wars, terrorism, car and other traffic accidents, job-related injuries, sports injuries, violent crimes, domestic accidents, child abuse and domestic violence; or by blunt objects that go through the skull, for example gunshots, wounds, etc.

TBI is one of the leading causes of death and disability worldwide. Estimates indicate that around 10 million people suffer TBI worldwide. Therefore, finding a course of treatment that prevents further damages after the injury is critical (SHARMA D, VAVILALA MS. Perioperative management of adult traumatic brain injury. Anesthesiology Clinics. 30:333-46. 2012; HYDER, A.A. et al. The impact of traumatic brain injuries: a global perspective. NeuroRehabilitation 22(5):341-353. 2007). To ensure recovery, metabolism should be normalized as soon as possible after the trauma to prevent subsequent functional deficits (KRISHNA, G. et al. 7,8 dihydroxyflavone facilitates the action exercise to restore plasticity and functionality: Implications for early brain trauma recovery. Biochimica et Biophysica Acta - Molecular Basis of Disease. (6):1204-1213. 2017).

Physical, behavioral and/or mental changes resulting from TBI will depend on the area that was damaged. Injuries include focal and diffuse brain damage. Focal damage is limited to a small area of the brain. Focal damage is more frequent on the spot where the head was hit by a blunt object or an object like a bullet entered the brain. Diffuse damage extends throughout the brain. While immediate treatment of head injuries has improved consistently in the last few decades, persistent effects like disabilities are still likely after moderate and severe TBI. It is now known that in TBI the primary injury triggers a secondary brain injury process in the following hours and days.

Hence, it could be said that two important pathophysiological processes contribute to the brain injury after the trauma, i.e. the primary injury where the damage is the direct result of the mechanic impact and the secondary injury that is triggered immediately after the trauma due to new cellular damage resulting from the effects of primary injuries that evolve along a period of hours and days following the initial damage (MAAS, A. I., STOCCHETTI, N. AND BULLOCK, R. Moderate and severe traumatic brain injury in adults. The Lancet Neurology, 7, 728-741. 2008). During this second phase, the main known mechanism of pathogenesis in cell damage is mostly activated by the release of neurotransmitters, calcium homeostasis, oxidative stress, and the inflammation and permeation of the blood-brain barrier (ANGELONI, C. et al. Traumatic brain injury and NADPH oxidase: a deep relationship. Oxidative Medicine and Cellular Longevity. 370312. 2015).

According to diagnosis criteria, TBI has one or more of the following characteristics: changes in the level of consciousness; memory decline; confusion associated to disorientation; neurological signs, like brain injuries that can be seen in neuroimaging, new or worsened convulsive seizures, visual field deficit and hemiparesis. Although some symptoms may emerge immediately after the injury, others may evolve with time consistently with anatomic changes occurred in neural substrates after the injury.

TBI survivors may experience a broad range of deficits. Sensory-motor and cognitive decline are also some of the common consequences of these injuries. Sensory-motor decline includes elements of paresis, postural imbalance/balance impairment and gait disorders, and early acute startle response (SR) impairment. TBI may cause bradykinesia, oscillation abnormalities and a deteriorated reaction time. The early balance impairment is a predictor of a poor prognosis after a TBI. Sensory-motor problems may improve with time; although severe deficits may persist during the first 1-2 years after the trauma. In the cognitive domain, there may be memory disorders, attention deficit and a slowdown in the information processing rate. More severe TBI cause greater and more lasting deficits than mild to moderate TBI. Currently, there are no appropriate courses of treatment to prevent TBI long term effects.

The primary phase of TBI describes the immediate damage to the brain tissue due to contusions or hypoxia caused by the mass effect. Primary injuries in TBI can only be reduced through better prevention. Secondary injuries begin after the trauma and are underlying to TBI-related functional deficits. They occur later through mechanisms like reperfusion injury, late onset cortical edema, breakdown of the blood-brain barrier, glutamatergic storm and local electrolyte imbalance. These alterations alone can cause ROS-mediated neurodegeneration, through the release of calcium, glutamate toxicity, lipid peroxidation and mitochondrial dysfunction. Said secondary injury may occur adjacent to the brain site of the alleged primary injury, which raises the probability of unexpected spread of the damaged area in the following months.

Key factors contributing to this second brain damage cascade include: excitatory aminoacids like glutamate, Ca ++ homeostasis and reactive oxygen species (ROS) (Kluger et al. 2004, Gaetz et al. 2004). Mitochondria are some of the cell organelles affected by secondary brain injuries, particularly the potential collapse of the mitochondrial membrane (D i) seems to play a central role among factors leading to brain cell death due to secondary brain injuries and neurodegenerative diseases (Kluger et al. al. 2004; Gaetz et al.2004).

Currently, TBI is considered an unsatisfied healthcare need. Therefore, developing effective therapeutic interventions to protect the brain and promote restoration after a traumatic brain injury is a particularly urgent task.

JM-20 (3-ethoxycarbonyl-2-methyl-4-(2-nitrophenyl)-4,11-dihydro-1H-pyridol[2,3-b][1,5] benzodiazepine), its derivatives and pharmaceutical compounds containing it were protected for the specific treatment of neurodegenerative disorders, with cognitive decline, Parkinson's disease and neuropathic pain, associated with aging under WO/2017/190713. However, there are no prior studies using JM-20 as a therapy for TBI. Inventors of this invention have surprisingly found that JM-20 has useful actions for the treatment of TBI.

Brief Description of the Invention

This invention refers to the use of JM-20 to reduce and/or reverse TBI adverse effects. It specifically, helps increase patients- rate of recovery after a TBI, including their sensory-motor, cognitive and spatial memory recovery.

Another aspect of this invention refers to a treatment method to reduce and/or reverse TBI, administering patients a therapeutically effective dose of JM-20 to reverse behavioral, morphological and biochemical changes.

BRIEF DESCRIPTION OF THE DRAWINGS

Figure 1. Effects of TBI and JM-20 treatment on latency to fall during rotarod test.
Figure 2. Effects of TBI and JM-20 treatment on crossings events during the open field test.
Figure 3. Effects of TBI and JM-20 treatment on rearing events during the open field test.
Figure 4. Effects of TBI and JM-20 treatment on immobility time during the open field test.
Figure 5. Effects of TBI and JM-20 treatment on time to complete the beam-walk test.
Figure 6. Effects of TBI and JM-20 treatment on brain water content.
Figure 7. Effects of TBI and JM-20 treatment on p-Akt expression in cortex by Western blotting analysis.
Figure 8. Effects of TBI and JM-20 treatment on p-Akt expression in hippocampus by Western blotting analysis.
Figure 9. Effects of TBI and JM-20 treatment on iba-1 expression in cortex by Western blotting analysis.
Figure 10. Effects of TBI and JM-20 treatment on iba-1 expression in hippocampus by Western blotting analysis.
Figure 11. Effects of TBI and JM-20 treatment on GFAP expression in cortex by Western blotting analysis.
Figure 12. Effects of TBI and JM-20 treatment on GFAP expression in hippocampus by Western blotting analysis.

Detailed Description of the Invention

Inventors of the present invention surprisingly found that the use of the JM20 compound has biological activity in the treatment of TBI.

When assessing the effects of administering JM-20 on the following parameters in animal models after a TBI, namely: motor performance; cerebral edema; astrocyte reactivity; microglial activation; and pro-survival pathway activation, it proved to be effective in reducing and, in some cases, reversing TBI adverse effects, including behavioral, morphological and biochemical changes.

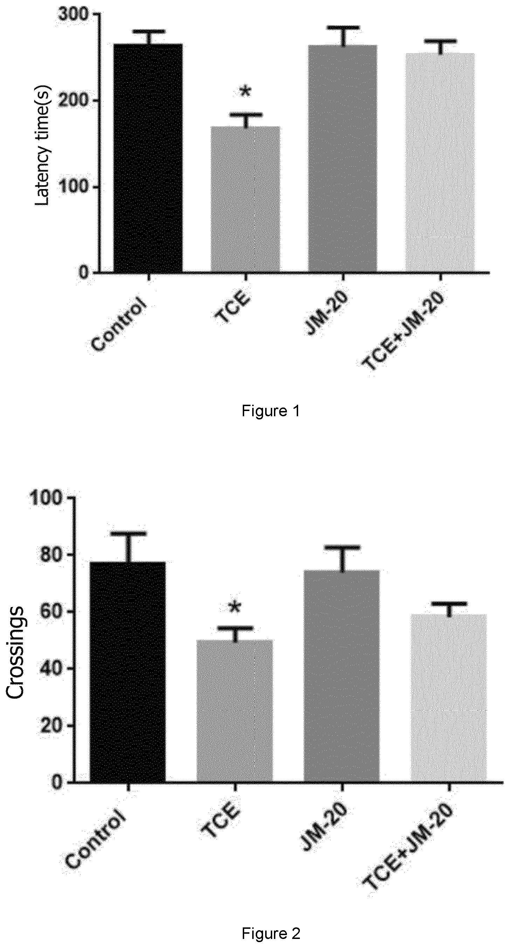

During behavioral tests, it was observed that animals who suffered a TBI showed a reduction of the first fall latency time on the rotarod test in contrast with the other groups ($p < 0.05$). In Figure 1 the values are expressed as mean $\pm$ SEM (n = 8-11, per group), where (*) are the differences of the control, JM-20 and TBI + JM-20 groups, $p < 0.05$ (by Kruskal-Wallis test followed by the Dunn post hoc test).

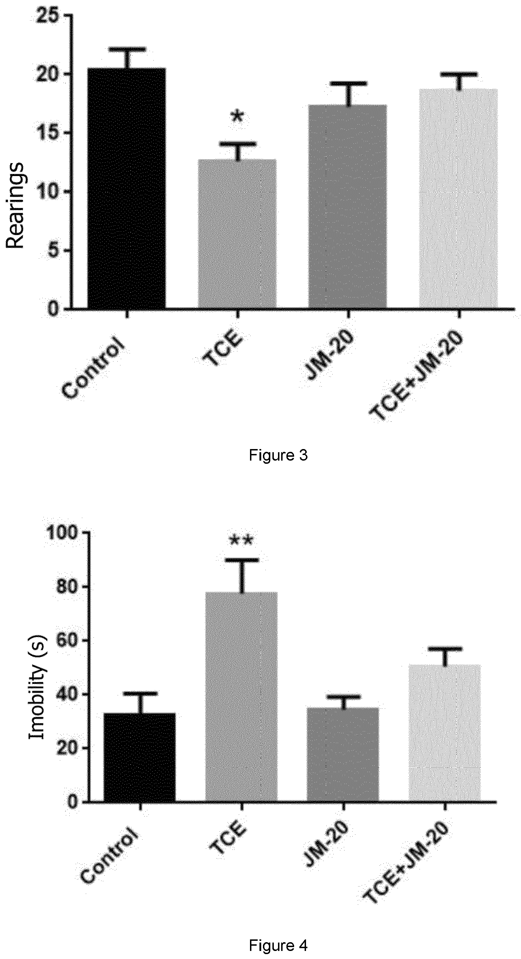

They also had a lower number of crossings and a longer immobility time during the open field test in comparison with the control group ($p < 0.05$ y $p < 0.01$). In Figure 2 the values are expressed as mean $\pm$ SEM (n = 8-11, per group) where (*) are the differences of the control groups, $p < 0.05$ (one-way ANOVA followed by the Tukey post hoc test). While in Figure 3, the data are reported as mean $\pm$ SEM (n = 8-11, per group), where (*) are the differences between the control and TBI + JM-20 group, $p < 0.05$ (by one-way ANOVA followed by the Tukey post hoc test). Additionally, a higher number of rearings was observed in comparison with the control and the TBI + JM-20 group ($p < 0.05$). In Figure 4 the data are reported as mean $\pm$ SEM (n = 8-11, per group). (**) Different from the control, $p < 0.01$ (by one-way ANOVA followed by the Tukey post hoc test).

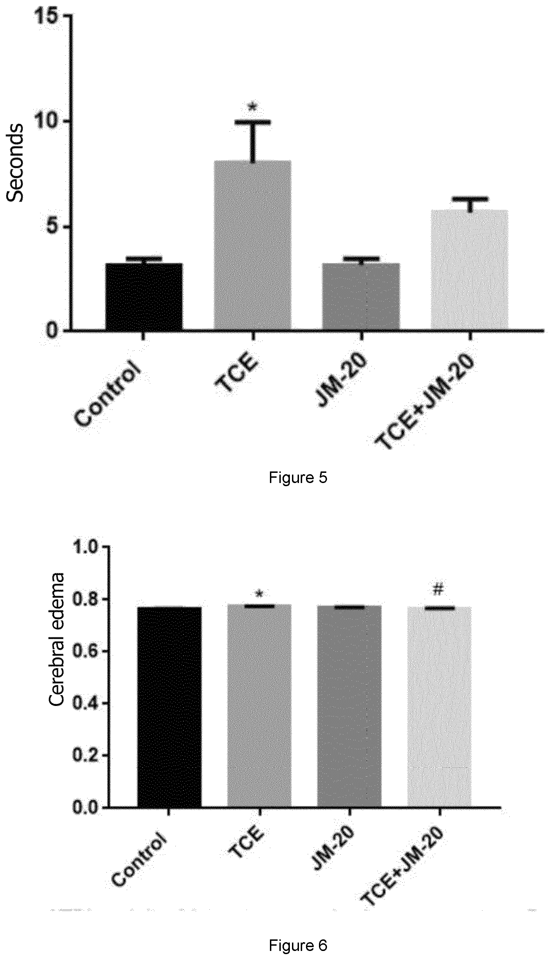

In the beam-walking test, rats in the TBI group took longer to complete the course than the control and JM-20 groups (p <0.05). In figure 5 the data are expressed as mean ± SEM (n = 6, per group). (*) Different from the control and JM20 group, p <0.05 (by one-way ANOVA followed by the Tukey post hoc test.)

The content of water in the brain increased in the TBI group in contrast with the control and TBI + JM-20 groups (p <0.05). In Figure 6 the data are reported as mean ± SEM (n = 6). (*) Different from the control, # different from the TBI group p <0.05 (by one-way ANOVA followed by the Tukey post hoc test).

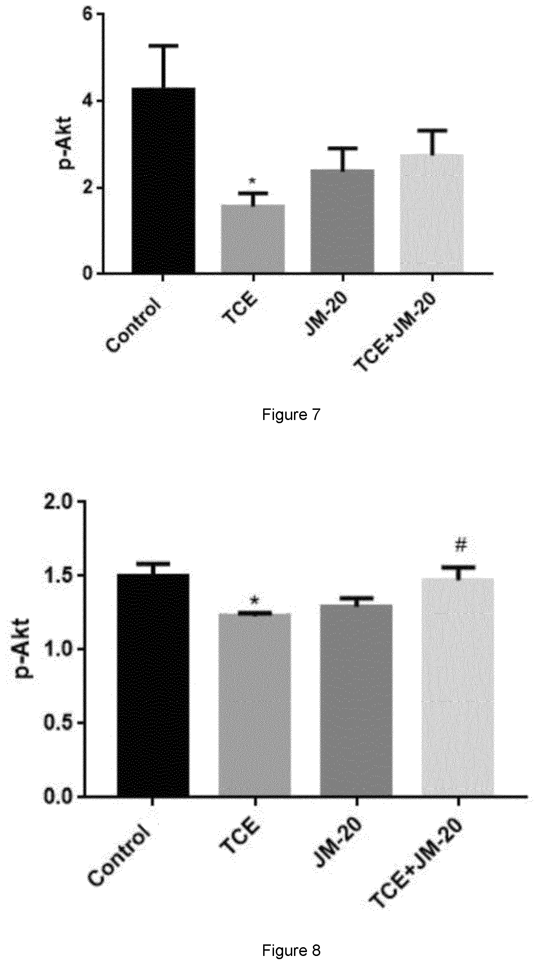

In the Western blotting, the p-Akt expression decreased in TBI groups, both in the cerebral cortex and the hippocampus in comparison with the control group (p <0.05), and the hippocampus in comparison with the control and TBI + JM-20 groups (p <0.05). In Figure 7, the data are reported as mean ± SEM (n = 6). (*) In contrast with the control, p <0.05 (by unidirectional ANOVA followed by the Tukey post hoc test). While in Figure 8 the data are reported as mean ± SEM (n = 6). (*) Different from the control, p <0.05; # different from TBI, p <0.05 (by one-way ANOVA followed by the Bonferroni post hoc test).

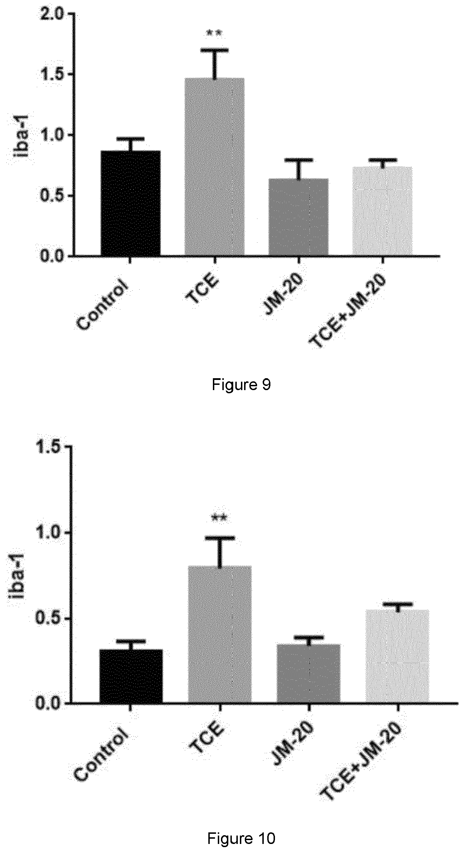

The iba-1 expression increased in TBI groups, both in the cerebral cortex in comparison with all the groups (p <0.01), and in the hippocampus in comparison with the control group (p <0.01). In Figure 9, the data are reported as mean ± SEM (n = 6). (**) Different from the control, JM-20 and TBI + JM-20 p <0.01 (by one-way ANOVA followed by the Newman-Keuls post hoc test). While in figure 10, the data are reported as mean ± SEM (n = 6). (**) Different from the control, p <0.01 (by one-way ANOVA followed by the Tukey post hoc test).

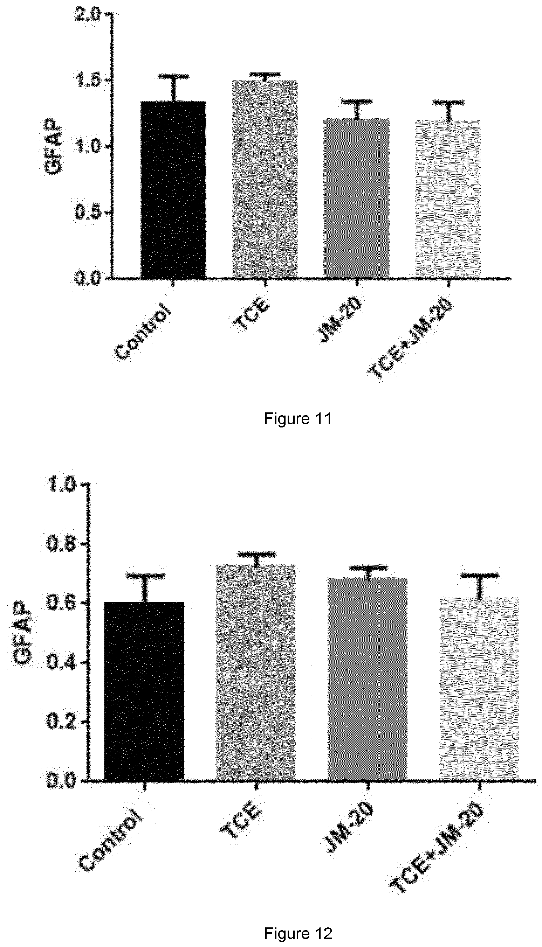

The GFAP expression showed no changes in any of the groups. Both in Figures 11 and 12, the data are reported as mean ± SEM (n = 6).

EXAMPLES

Animals

Male Wistar rats (200-250 g), with an average age of 60 days were used, conditioned in boxes with food and water at will. Procedures were performed pursuant to the rules of the Animal Ethics and Welfare Committee UFSM (9426190418).

Experimental Design

TBI Induction

Performance tests

TBI was induced through the weight-drop model (MANNIX, R. et al. Chronic Gliosis and Behavioral Deficits in Mice Following Repetitive Mild Traumatic Brain Injury. Journal of Neurosurgery. 2014) A 54 g weight was used released from a 100 cm height freely falling on the animals head. Animals were suspended on aluminum foil, with small cuts to ensure it would break with the impact of the weight allowing for the sudden acceleration of the movement, thus tearing the aluminum foil and falling over a sponge. TBI-induced animals were previously anesthetized with 2% isoflurane. In addition, topical lidocaine was applied to the animals' head to minimize posttraumatic pain (MYCHASIUK, R. et al. A Novel Model of Mild Traumatic Brain Injury for Juvenile Rats. Journal of Visualized Experiments. 2014)

Euthanasia

Animals were beheaded by shearing and their brains were immediately extracted and their hippocampi and cortices were dissected.

Performance Tests

Rotarod

Animals were trained for 5 minutes before the TBI so they could adapt to the apparatus. The testing session comprised 5 trials and concluded when animals fell off the rotor (3,7 cm diameter, velocity 25-30 rpm) or after the cutoff time, i.e. 300 s. (Whishaw et al., 2003). The first fall latency was analyzed.

Open Field Test

Animals were placed in the central area of an open field (56 cm diameter) that had its surface divided into equal parts. The duration of the test was 300 seconds. The number of times animals walked around the quadrants, the number of their exploratory responses (rearings) and their immobility time were analyzed.

Beam-Walking Test

The beam-walking test (HAUSSER, N. et al. Detecting Behavioral Deficits in Rats After Traumatic Brain Injury. J. Vis. Exp. (131), e56044, doi:10.3791/56044. 2018.) consists in animals walking on a hanging wooden beam (2.5 cm wide and 100 cm long) to reach a black wooden box placed at the end of the apparatus. First, the rats were placed in the wood box for one minute for acclimation. Shortly after, they were placed on the other end and encouraged to walk along the beam to reach the opposite end. Three attempts were analyzed. The rats were trained prior to the TBI for familiarization with the apparatus. The day of the test, the times of each attempt were recorded and their values were averaged.

Cerebral Edema

The cerebral edema was determined measuring the water content of the brain using the wet-dry method described by Chen et al (2014) (CHEN, W. et al. Neuroprotective effect of allicin against traumatic brain injury via Akt/endothelial nitric oxide synthase pathway-mediated antiinflammatory and anti-oxidative activities. Neurochemistry International. 68:28-37. 2014) Twenty-four hours after the TBI, the animals were sacrificed and their brains were rapidly removed and weighed to determine their wet weight. Then they were dried in the oven at 100 ° C for 48 hours, the tissues were then weighted again until they were dry. The water content of the brain was calculated using the following formula:

$$\% \ H2O = (1 - \text{dry weight} / \text{wet weight})$$

Western blotting

The Western blot test was performed according to Gerbatin et al (2017) with some adjustments (GERBATIN, R.D.R. et al. Guanosine Protects Against Traumatic Brain Injury-Induced Functional Impairments and Neuronal Loss by Modulating Excitotoxicity, Mitochondrial Dysfunction, and Inflammation. Molecular Neurobiology. 54(10):7585-7596. 2017.). The hippocampus and cerebral cortex tissue samples were lysed in the RIPA (radioimmunoprecipitation assay) and centrifuged for 20 minutes at 12.700 x g and 4 ° C. The protein concentration of each sample was determined by the bicinchonininc acid protein assay (Thermo Fisher Scientific). The samples (30 $\mu$g of protein) were subjected to SDS-polyacrylamide gel electrophoresis at 4-12% and transferred to a nitrocellulose membrane using the Trans-Blot® Turbo ™ transference system and the protein load was confirmed by Ponceau S solution (Sigma Aldrich - P7170). After the specific blockade, the transferences were incubated for one night at 4 ° C with one rabbit anti-Iba-1 ionized calcium-binding adaptor molecule (1: 400; Santa Cruz Biotechnology, Santa Cruz, CA, EE. UU.), rabbit anti-glial fibrillary acidic protein (GFAP) (1: 1000; Dako), Phospho-Akt (1: 1000; Cell signaling).

The mouse anti-$\beta$-actin antibody (1: 10,000, Santa Cruz Biotechnology, Santa Cruz, CA, EE. UU.) was dyed as an additional control to the protein load. After the primary antibody was incubated, the membranes were rinsed in TBS-T (TBS + Tween 20 at 0.1%) twice at room temperature for 10 minutes and incubated with anti-rabbit (Sigma Aldrich-A6154) or anti-mouse (Santa Cruz Biotechnology - sc -2005) secondary antibodies conjugated to horseradish peroxidase (HRP) (1: 5000) for two hours at room temperature. The bands were visualized by enhanced chemiluminescence using ECL Western Blotting substrate (Pierce ECL, BioRad) and the signals were registered with the photodocumentation system ChemiDoc XRS + (BioRad). Then the bands were quantified using the Image Lab software (Bio-Rad).

**Claims**

1. Use of the JM20 compound in the treatment of traumatic brain injury (TBI).

2. The compound according to claim 1 above, where the treatment improves patients' recovery rate after a TBI.

3. The compound according to claim 1 above, where the treatment improves patients' sensory-motor recovery after a TBI.

4. The compound according to claim 1 above, where the treatment improves patients' cognitive recovery after a TBI.

5. The compound as described in claim 1 above, where the treatment improves patients' spatial memory after a TBI.

6. A method of treatment to reduce and/or reverse TBI in patients including administering them a therapeutically effective dose of JM20.

7. The treatment method described in claim 6 above, where the treatment is effective in reducing and/or reversing behavioral, morphological and biochemical changes.

Figure 1

Figure 2

Figure 3

Figure 4

Figure 5

Figure 6

Figure 7

Figure 8

Figure 9

Figure 10

Figure 11

Figure 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2017190713 A

### Non-patent literature cited in the description

- **SHARMA D ; VAVILALA MS.** Perioperative management of adult traumatic brain injury. *Anesthesiology Clinics,* 2012, vol. 30, 333-46
- **HYDER, A.A. et al.** The impact of traumatic brain injuries: a global perspective. *NeuroRehabilitation,* 2007, vol. 22 (5), 341-353
- **KRISHNA, G. et al.** 7,8 dihydroxyflavone facilitates the action exercise to restore plasticity and functionality: Implications for early brain trauma recovery. *Biochimica et Biophysica Acta - Molecular Basis of Disease,* 2017, vol. 6, 1204-1213
- **MAAS, A. I. ; STOCCHETTI, N. ; BULLOCK, R.** Moderate and severe traumatic brain injury in adults. *The Lancet Neurology,* 2008, vol. 7, 728-741
- **ANGELONI, C. et al.** Traumatic brain injury and NADPH oxidase: a deep relationship. *Oxidative Medicine and Cellular Longevity,* 2015, 370312
- **MANNIX, R. et al.** Chronic Gliosis and Behavioral Deficits in Mice Following Repetitive Mild Traumatic Brain Injury. *Journal of Neurosurgery,* 2014
- **MYCHASIUK, R. et al.** A Novel Model of Mild Traumatic Brain Injury for Juvenile Rats. *Journal of Visualized Experiments,* 2014
- **HAUSSER, N. et al.** Detecting Behavioral Deficits in Rats After Traumatic Brain Injury. *J. Vis. Exp.,* 2018, (131), e56044
- **CHEN, W. et al.** Neuroprotective effect of allicin against traumatic brain injury via Akt/endothelial nitric oxide synthase pathway-mediated antiinflammatory and anti-oxidative activities. *Neurochemistry International,* 2014, vol. 68, 28-37
- **GERBATIN, R.D.R. et al.** Guanosine Protects Against Traumatic Brain Injury-Induced Functional Impairments and Neuronal Loss by Modulating Excitotoxicity, Mitochondrial Dysfunction, and Inflammation. *Molecular Neurobiology.,* 2017, vol. 54 (10), 7585-7596